# EUROPEAN PATENT APPLICATION

(11) **EP 2 985 023 A1**
(43) Date of publication of application: **17.02.2016**
(21) Application number: 15186114.3
(22) Date of filing: 30.07.2012
(51) Int. Cl.: A61K 31/4365, C07C 45/63, C07D 495/04

(54) **PROCESS FOR PREPARING PRASUGREL**

(30) Priority: 28.07.2011 EP 11175721; 29.07.2011 US 201161574248 P; 10.01.2012 EP 12150675
(62) Divisional of application: 12740371.5
(71) Applicant: Laboratorios Lesvi, S.L., 08970 Sant Joan Despi (ES)
(72) Inventor: NEWADKAR, Ravindranath Vishnu, 400610 THANE (IN); PURUSHOTTAM JOSHI, Anil, 400602 THANE (IN); RAGHUNATH BENDRE, Samir, 400605 THANE (IN); HEMANT JERE, Deepak, 421304 THANE (IN); DALMASES BARJOAN, Pere, 08970 SANT JOAN DESPI (ES); NAVARRO MUÑOZ, Isabel, 08970 SANT JOAN DESPI (ES); HUGUET CLOTET, Juan, 08970 SANT JOAN DESPI (ES)
(74) Representative: Oficina Ponti, SLP

(57) **Abstract**

The present invention is directed to an improved process for preparing, prasugrel and maleate salt of Prasugrel and, optionally other pharmaceutically acceptable salts from, prasugrel and said maleate salt, in high yields and purity, which can be used at industrial scale.

The process of the present invention comprises the steps of bromination, condensation, acetylating and optionally converting into maleate salt and, if desired, conversion into another pharmaceutically acceptable salt from it. The present process is advantageous in terms of productivity, efficacy, purity and also prevents the use of toxic substances.

## Description

### FIELD OF THE INVENTION

The present invention is directed to an improved process for preparing, prasugrel and maleate salt of prasugrel and, optionally other pharmaceutically acceptable salts from prasugrel and said maleate salt, in high yields and purity, which can be used at industrial scale.

The maleate salt of prasugrel exhibits excellent oral absorption, metabolism into the active compound and activity in inhibition of platelet aggregation, low toxicity, and further excellent storage and handling stability. It is useful as a prophylactic or therapeutic agent for thrombosis or embolism.

### BACKGROUND OF THE INVENTION

Prasugrel is an orally-active P2Y12 purinoreceptor antagonist and the lead in a series of tetrahydrothienopyridine derivatives, developed by Sankyo (Daiichi Sankyo) and Ube Industries as a platelet aggregation inhibitor for the prophylaxis and therapy of thrombosis. Its chemical name is 2-acetoxy-5-(alpha-cyclopropylcarbonyl-2-fluorobenzyl)-4,5,6,7-tetrahydrothieno[3,2-c]pyridine.

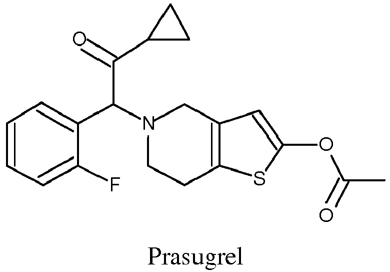

The maleate salt of prasugrel exhibits excellent oral absorption, metabolism into the active compound and activity in inhibition of platelet aggregation, low toxicity, and further excellent storage and handling stability. It is useful as a prophylactic or therapeutic agent for thrombosis or embolism.

Prasugrel was first disclosed by European patent number EP 0 542 411 by Sankyo Company Limited and Ube Industries. EP 0 542 411 disclosed the preparation of prasugrel by reacting cyclopropyl-2-fluorobenzyl ketone with bromine in carbon tetrachloride to obtain 2-fluoro-alpha-cyclopropyl carbonyl benzyl bromide, as described in scheme 1. The bromide intermediate was mixed with 5,6,7,7a-tetrahydrothieno[3,2]pyridine-2(4H)-one in DMF in the presence of anhydrous potassium carbonate to yield 5-(alpha-cyclopropyl carbonyl-2-fluorobenzyl)-2-oxo-2,4,5,6,7,7a-hexahydro-thieno[3,2c]pyridine, which was mixed further with DMF, acetic anhydride and sodium hydride to yield crude Prasugrel. Prasugrel was purified by column chromatography.

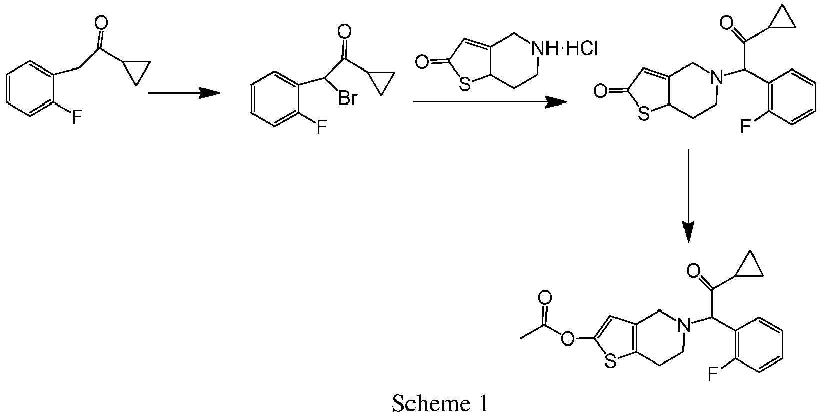

The process of Scheme 1 has been found to have several disadvantages when prasugrel is prepared at industrial scale, since environmental unfriendly reagents are used in the reaction medium and low yields are achieved.

EP1298132 B1 describes a process for the preparation of 2-fluoro-alpha-cyclopropyl carbonyl benzyl bromide wherein the bromation is carried out in a mixture of aqueous hydrogen halide or an alkali metal salt of aqueous hydrogen halide. Although more environmental friendly reagents were used in the reaction medium, the reaction temperature and the reaction time were very high, taking up to 5 days, for the reaction to be completed.

EP1298132 B1 describes a process for the preparation of 2-Acetoxy-5-([alpha]- cyclopropylcarbonyl-2-fluorobenzyl)-4,5,6,7-tetrahydrothieno[3,2-c]pyridine hydrochloride salt (the hydrochloride salt of prasugrel). The preparation of the hydrochloride salt of prasugrel was carried out by dropwise addition of concentrated hydrochloric acid to a solution of 2-Acetoxy-5-([alpha]- cyclopropylcarbonyl-2-fluorobenzyl)-4,5,6,7-tetrahydrothieno[3,2-c]pyridine in an inert solvent at elevated temperature (from 35 °C to 60 °C). The 2-Acetoxy-5-([alpha]- cyclopropylcarbonyl-2-fluorobenzyl)-4,5,6,7-tetrahydrothieno[3,2-c]pyridine used in the reaction was purified by column chromatography which is not feasible at industrial scale.

EP 1728794 B1 teaches a process for the preparation of 2-Acetoxy-5-([alpha]-cyclopropylcarbonyl-2-fluorobenzyl)-4,5,6,7-tetrahydrothieno[3,2-c]pyridine maleate salt (the maleate salt of prasugrel). The preparation of the prasugrel maleate salt is carried out by reacting cyclopropyl-2-fluorobenzyl ketone with N-bromosuccimide in carbon tetrachloride to obtain 2-fluoro-alpha-cyclopropyl carbonyl benzyl bromide. The bromide intermediate compound is mixed with 2-oxo-2,4,5,6,7,7a-hexahydrothieno[3,2-c]pyridine hydrochloride and potassium bicarbonate to yield 5-(alpha-cyclopropyl carbonyl-2-fluorobenzyl)-2-oxo-2,4,5,6,7,7a-hexahydro-thieno [3,2c]pyridine, which is mixed further with DMF, acetic anhydride and sodium hydride to yield Prasugrel. A solution of maleic acid acetone is added to the Prasugrel previuosly obtained to yield prasugrel maleate in very low yields. Moreover, said process uses environmental unfriendly agents such as carbon tetrachloride among others.

EP 2112155 B1 by Sandoz teaches the preparation of the hydrogensulphate salt of prasugrel by heating a mixture of prasugrel base or prasugrel maleate, sulphuric acid and acetone affording prasugrel hydrogensulphate in very low yields.

Moreover, WO2011/057592 by Zentiva teaches a process for the preparation of salts of prasugrel such as hydrobromide, hydroiodide, benzene sulphonate or cyclamate from prasugrel base. However, the yields of the overall process were very low.

Therefore, a process for obtaining Prasugrel and Prasugrel maleate or another pharmaceutically acceptable salt thereof in a high yield and purity, and using reactants more environmentally friendly than those used in the state of the art, and feasible at industrial scale, are still remained.

### BRIEF DESCRIPTION OF THE INVENTION

The present inventors have developed a process for the preparation of Prasugrel and Prasugrel maleate or other pharmaceutically acceptable salts thereof in good yields and a high purity. Advantageously, the improved process is applicable at industrial scale and uses less toxic reactants and solvents and thus is more environmentally friendly.

The first aspect of the present invention is to provide an improved process for the preparation of Prasugrel, and optionally the pharmaceutically acceptable salts thereof, comprising the following steps:
a) bromination of the compound of formula (V) 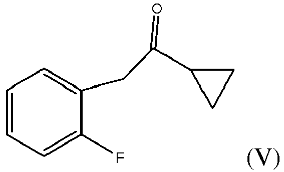 with liquid bromine in the presence of a polar protic solvent to obtain the compound of formula (IV) 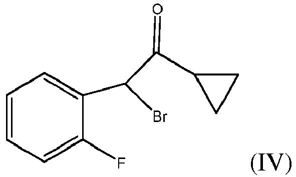
b) condensing the compound of formula (IV) with the compound of formula (III) 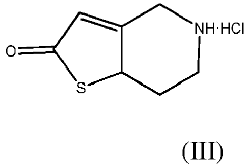 in the presence of a base in a polar aprotic solvent to obtain the compound of formula (II) 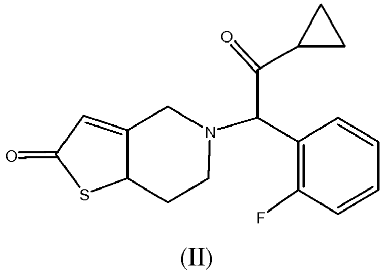
c) acetylating the compound of formula (II) in the presence of a polar aprotic solvent, an acetylating agent and a catalyst to obtain Prasugrel base of formula (I), 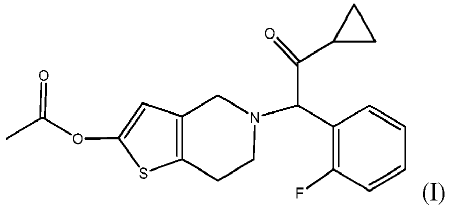
and, optionally, preparing the pharmaceutically acceptable salts thereof by either:
d) converting Prasugrel base of formula (I) with maleic acid in the presence of a polar aprotic solvent into Prasugrel maleate of formula (Ib) 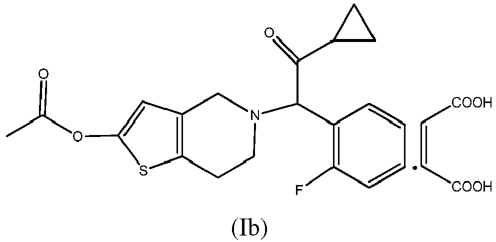 and, optionally, purifying prasugrel maleate, obtaining prasugrel base and, then converting prasugrel base into another pharmaceutically acceptable salt;
or either
e) converting Prasugrel base of formula (I) into a pharmaceutically acceptable salt of prasugrel.

In an embodiment of the present invention, prasugrel maleate of formula (Ib) is further purified and converted to prasugrel base and, afterwards, if desired, prasugrel base is converted into another pharmaceutically acceptable salt of the type H·X, by adding a solution of the conjugated acid of the desired salt in a polar organic solvent. Particularly, the hydrochloride salt of formula (Ia) is prepared by adding to the previous prepared prasugrel base a solution of HCl in a polar organic solvent or by adding to previous prepared prasugrel base a protic solvents or aprotic solvents in combination with at least one equivalent of a protic solvent compared to prasugrel and tumethylchlorosilane (TMS-Cl).

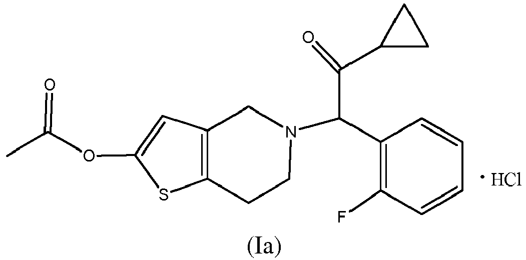

The process of the present invention does not require the use of chromatographic methods for purification, which are not feasible at industrial scale.

The second aspect of the present invention is to provide an improved process for the preparation of a compound of formula (IV): 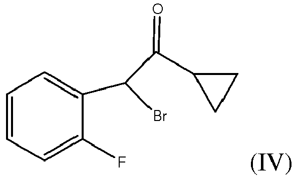 comprising a) bromination of the compound of formula (V): 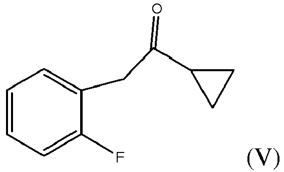 with liquid bromine in the presence of a polar protic solvent to obtain the compound of formula (IV).

The third aspect of the present invention is the use of the compound of formula (IV) obtained according to the second aspect of the present invention for preparing prasugrel base and salts thereof.

### DETAILED DESCRIPTION OF THE INVENTION

Unless defined otherwise, all technical and scientific terms used herein have the same meaning as commonly understood by one of ordinary skill in the art to which this invention belongs.

The term "Prasugrel" refers to 2-Acetoxy-5-([alpha]-cycloprpylcarbonyl-2-fluorobenzyl)-4,5,6,7- tetrahydrothieno[3,2-c]pyridine.

The term "PTSA" refers to paratoluene sulfonic acid.

The term "TMS-Cl" refers to trimethylsilyl chloride.

The term "IPA" refers to isopropyl alcohol.

The term "DMF" refers to dimethylformamide.

The term "DMAC" refers to dimethylacetamide.

The term "THF" refers to tetrahydrofuran.

The term "MIBK" refers to methyl isobutyl ketone.

The term "polar solvent" relates to a solvent in whose molecules there is either a permanent separation of positive and negative charges, or the centres of positive and negative charges do not coincide. Examples of polar solvents are alcohols, alkoxylated alcohols, aryloxylated alcohols and polyols, such as IPA, butanol, n-propanol, methanol, benzyl alcohol, 2-benzyloxyethanol, benzyl glycol, 1,2-butanediol, 1,3-butanediol, 1,4-butanediol, 2,3-butanediol, butylene glycol, butylene glycol proprionate, butyloctanol, cyclohexanedimethanol, 1,10-decanediol, diethoxydiglycol, dipropylene glycol, ethoxydiglycol, ethylene glycol, hexandiol, 1,2-hexanediol, 1,2,6-hexanetriol, hexylene glycol, isobutoxypropanol, isopentyldiol, 3-methoxybutanol, methoxybutanol, methoxyisopropanol, methoxymethylbutanol, pentylene glycol, 2-phenoxyethanol, 1-phenoxy-2-propanol, 2-phenyethanol, propanediol, propyl alcohol, propylene glycol, trimethyl-1,3-pentanediol, DMAC, THF, acetonitrile, benzyl benzoate, butoxyethyl acetate (regular), butyl acetate, *t*-butyl acetate, butyloctyl benzoate, dimethyl glutarate, dimethyl maleate, dipropyl oxalate, ethoxydiglycol acetate, ethyl acetate, ethylhexyl acetate, ethylhexyl benzoate, ethyl lactate, isopropyl acetate, methyl acetate, methyl hexyl ether, acetone, ethyl ketone, methyl tert butyl ether, ethyl acetate, methyl acetate, propyl acetate, isopropyl acetate, butyl acetate, isobutyl acetate, N-methylpyrrolidone, dichloromethane, acetone, dimehtyl sulfoxideor mixtures thereof.

The term "protic solvent" refers to any molecular solvent which contains dissociable H⁺. The molecules of such solvents can donate an H⁺.

The term "aprotic solvent" refers to any molecular solvent which cannot donate H⁺.

The term "polar protic solvent" relates to a polar solvent that is capable of exchanging protons with the reagents and that has a polarizable proton. Examples of protic polar solvents are alcohols, alkoxylated alcohols, aryloxylated alcohols and polyols, such as IPA, butanol, n-propanol, methanol, ethanol, benzyl alcohol, 2-benzyloxyethanol, benzyl glycol, 1,2-butanediol, 1,3-butanediol, 1,4-butanediol, 2,3-butanediol, butylene glycol, butylene glycol proprionate, butyloctanol, cyclohexanedimethanol, 1,10-decanediol, diethoxydiglycol, dipropylene glycol, ethoxydiglycol, ethylene glycol, hexandiol, 1,2-hexanediol, 1,2,6-hexanetriol, hexylene glycol, isobutoxypropanol, isopentyldiol, 3-methoxybutanol, methoxybutanol, methoxyisopropanol, methoxymethylbutanol, pentylene glycol, 2-phenoxyethanol, 1-phenoxy-2-propanol, 2-phenyethanol, propanediol, propyl alcohol, propylene glycol, trimethyl-1,3-pentanediol or mixtures thereof.

The term "polar aprotic solvent" relates to a polar solvent that is not capable of exchanging protons with the reagents and that has no polarizable proton. Examples of polar aprotic solvents are DMAC, THF, acetonitrile, methylene chloride, benzyl benzoate, butoxyethyl acetate (regular), butyl acetate, *t*-butyl acetate, butyloctyl benzoate, dimethyl glutarate, dimethyl maleate, dipropyl oxalate, ethoxydiglycol acetate, ethyl acetate, ethylhexyl acetate, ethylhexyl benzoate, ethyl lactate, isopropyl acetate, methyl acetate, methyl hexyl ether, acetone, ethyl ketone, methyl ethyl ketone, methyl isobutyl ketone, methyl tert butyl ether, ethyl acetate, methyl acetate, propyl acetate, isopropyl acetate, butyl acetate, isobutyl acetate, N-methylpyrrolidone, dichloromethane, acetone, dimethyl sulfoxide or mixtures thereof.

Other suitable aprotic solvents are hexane, 1,4-dioxane, chloroform, diethyl ether, toluene and xylene.

The term "catalyst" refers to a substance that increases the rate of a reaction without being consumed in the process. Examples of catalysts are PTSA, methane sulfonic acid, ethane sulfonic acid, benzene sulfonic acid, 1-naphthalene sulfonic acid, 1,5-naphthalenedisulfonic acid, sulphuric acid, triethylamine, .N-methylmorfoline, potassium carbonate, diisopropylethylamine and pyridine

The term "ammonia derivate" include triethyl amine, dicyclohexylamine, N,N-diisopropylethylamine, methanolic ammonia and other compounds containing NH₃.

The term "metal carbonate" includes metal carbonates and metal bicarbonates. Examples of "metal carbonates" are sodium carbonate, sodium bicarbonate, potassium bicarbonate, potassium carbonate, lithium bicarbonate, lithium carbonate

The term "purification" refers to the process wherein a purified drug substance can be obtained. The term "industrial purification" refers to purifications which can be carried out on an industrial scale which are well known by the skilled person in this field such as solvent extraction, filtration, slurring, washing, phase separation, evaporation, centrifugation or crystallisation.

As used herein, the term, "solvent extraction" refers to the process of separating components of a mixture by using a solvent which possesses greater affinity for one component, and may therefore separate said one component from at least a second component which is less miscible than said one component with said solvent.

The term "filtration" refers to the act of removing solid particles greater than a predetermined size from a feed comprising a mixture of solid particles and liquid. The expression "filtrate" refers to the mixture less the solid particles removed by the filtration process.

As used herein, the term "slurring" refers to any process which employs a solvent to wash or disperse a crude product.

As used herein, the term "washing" refers to the process of purifying a solid mass (e.g., crystals) by passing a liquid over and/or through the solid mass, as to remove soluble matter. The process includes passing a solvent, such as distilled water, over and/or through a precipitate obtained from filtering, decanting, or a combination thereof. For example, in one embodiment of the invention, washing includes contacting solids with solvent or solvent mixture, vigorously stirring (e.g., for two hours), and filtering. The solvent can be water, can be an aqueous solvent system, or can be an organic solvent system. Additionally, the washing can be carried out with the solvent having any suitable temperature. For example, the washing can be carried out with the solvent having a temperature between about 0 °C and about 100 °C.

The term "phase separation" refers to a solution or mixture having at least two physically distinct regions.

The term "evaporation" refers to the change in state of solvent from liquid to gas and removal of that gas from the reactor. Generally gas is removed by vacuum applied across the membrane. Various solvents may be evaporated during the synthetic route disclosed herein. As known to those of skill in the art, each solvent may have a different evaporation time and/or temperature.

The term "crystallization" refers to any method known to a person skilled in the art such as crystallization from single solvent or combination of solvents by dissolving the compound optionally at elevated temperature and precipitating the compound by cooling the solution or removing solvent from the solution or both. It further includes methods such as solvent/antisolvent or precipitation.

The first aspect of the present invention is to provide an improved process for the preparation of Prasugrel and, optionally a pharmaceutically acceptable salt thereof, comprising the following steps:
a) bromination of the compound of formula (V) 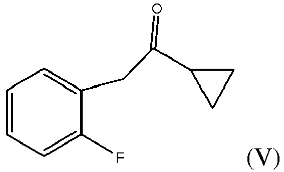 with liquid bromine in the presence of a polar protic solvent to obtain the compound of formula (IV)

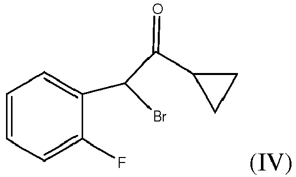

Suitable polar protic solvent include alcohols, alkoxylated alcohols, aryloxylated alcohols and polyols, such as IPA, butanol, n-propanol, methanol, ethanol, benzyl alcohol, 2-benzyloxyethanol, benzyl glycol, 1,2-butanediol, 1,3-butanediol, 1,4-butanediol, 2,3-butanediol, butylene glycol, butylene glycol proprionate, butyloctanol, cyclohexanedimethanol, 1,10-decanediol, diethoxydiglycol, dipropylene glycol, ethoxydiglycol, ethylene glycol, hexandiol, 1,2-hexanediol, 1,2,6-hexanetriol, hexylene glycol, isobutoxypropanol, isopentyldiol, 3-methoxybutanol, methoxybutanol, methoxyisopropanol, methoxymethylbutanol, pentylene glycol, 2-phenoxyethanol, 1-phenoxy-2-propanol, 2-phenyethanol, propanediol, propyl alcohol, propylene glycol, trimethyl-1,3-pentanediol. Among them, alcohols such as methanol, butanol or n-propanol are preferred, still more preferably methanol.

Preferably, the reaction was carried out in methanol, and the addition of a copper catalyst was not required to for the reaction to take place. The reaction temperature range was from 15 to 40°C to avoid undesirable by-products and also the multi-bromination of the compound of formula (V). Preferably, the reaction temperature range was from 20 to 32 °C. Most preferably, between 23 and 30 °C. The molar ratio of liquid bromine to compound of formula (V) may be from 1 to 2. Preferably, the molar ratio is from 1.1 to 1.5.

Advantageously the obtained yield was higher without the presence of a Cu-catalyst. Moreover, the yield obtained was above 94 %, higher than the yields disclosed in the prior art. The obtained purity was as high as 95.76 %.The examples below disclose more details about the reaction parameters, reaction methodology and work-up process.

The compound (IV) obtained may be further purified by conventional methods. Preferably, compound (IV) is purified by extraction with an organic solvent such as ethyl acetate or toluene.
b) condensing the compound of formula (IV) with a compound of formula (III) in the presence of a base and an aprotic solvent to obtain the compound of formula (II):

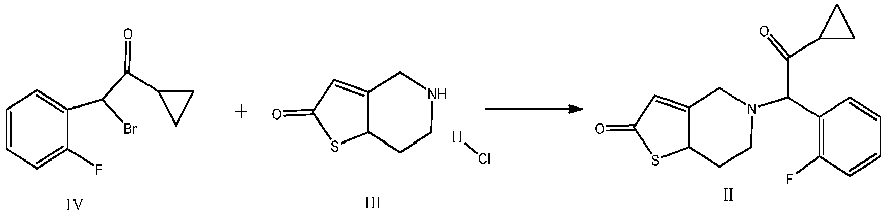

Suitable polar aprotic solvents include glyceryl esters, polymeric ethers, ketones and mixtures thereof, DMAC, THF, acetonitrile, methylene chloride, benzyl benzoate, butoxyethyl acetate (regular), butyl acetate, *t*-butyl acetate, butyloctyl benzoate, dimethyl glutarate, dimethyl maleate, dipropyl oxalate, ethoxydiglycol acetate, ethyl acetate, ethylhexyl acetate, ethylhexyl benzoate, ethyl lactate, isopropyl acetate, methyl acetate, methyl hexyl ether, acetone, ethyl ketone, methyl ethyl ketone, methyl isobutyl ketone, methyl tert butyl ether, ethyl acetate, methyl acetate, propyl acetate, isopropyl acetate, butyl acetate and isobutyl acetate. The preferred solvents are acetonitrile, methyl ethyl ketone and THF.

Other suitable aprotic solvents are hexane, 1,4-dioxane, chloroform, diethyl ether, toluene and xylene. The preferred solvent is toluene.

Suitable bases include, such as metal hydroxides, such as sodium hydroxide and potassium hydroxide, metal carbonates, such as sodium carbonate and potassium bicarbonate, ammonia derivatives such as triethyl amine, dicyclohexylamine, N,N-diisopropylethylamine, methanolic ammonia. Among them ammonia derivates and metal carbonates are preferred. More preferably, methanolic ammonia ad sodium carbonate.

The condensation reaction is carried out over a range of temperatures from -10 to 50°C.

In a specific embodiment the amount of ammonia derivative employed is not critical, and generally it can be from an equimolar amount of 2 to 3 with respect to the starting material of formula (IV). The condensation reaction can take place over a range of temperatures from -5 to 0°C. After the reaction is completed, the reaction mass can be extracted in ethyl acetate dried over sodium sulphate and concentrated under vacuum at 45-50°C. Toluene can also be used for the extraction. Advantageous results were obtained with acetonitrile as solvent and slow addition of methanolic ammonia at -5 to 0°C. Moreover, extractions carried out at a pH ranging from 6 to 8, preferably around 7 (7±0.5) yield the best results and the highest purity. Surprisingly, the reaction media acetonitrile/methanolic ammonia gives a yield as high as 95.4% and a purity as high as 88.6%.

In another specific embodiment the amount of metal carbonate employed is not critical, and generally it can be from an equimolar amount of 2 to 3 with respect to the starting material of formula (IV). The condensation reaction can take place over a range of temperatures from 15 to 30°C. Advantageous results were obtained with methyl ethyl ketone. Surprisingly, the reaction media sodium carbonate/methyl ethyl ketone gives a yield as high as 80% and a purity as high as 84%.
c) acetylating the compound of formula (II) in presence of an acetylating agent such as acetic anhydride, acetyl halides or acetates such as isopropenyl acetate, an aprotic solvent and a catalyst to obtain Prasugrel base of formula (I).

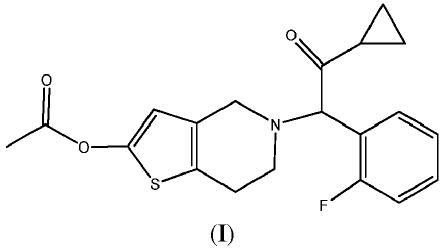

The preferred acetylating agents to obtain Prasugrel are acetates such as isopropenyl acetate, acetyl chloride, acetic anhydride or methyl acetate.

Suitable polar aprotic solvents are DMAC, THF, acetonitrile, methylene chloride, benzyl benzoate, butoxyethyl acetate (regular), butyl acetate, *t*-butyl acetate, butyloctyl benzoate, dimethyl glutarate, dimethyl maleate, dipropyl oxalate, ethoxydiglycol acetate, ethyl acetate, ethylhexyl acetate, ethylhexyl benzoate, ethyl lactate, isopropyl acetate, methyl acetate, methyl hexyl ether, acetone, ethyl ketone, methyl ethyl ketone, methyl isobutyl ketone, methyl tert butyl ether, ethyl acetate, methyl acetate, propyl acetate, isopropyl acetate, butyl acetate, isobutyl acetate, N-methylpyrrolidone, dichloromethane, acetone, dimethyl sulfoxide or mixtures thereof. Among them, acetonitrile, methylene chloride and isopropyl acetate are preferred.

Other suitable aprotic solvents are hexane, 1,4-dioxane, chloroform, diethyl ether, toluene and xylene. Preferably, the solvent is toluene.

Suitable catalysts are PTSA, methane sulfonic acid, ethane sulfonic acid, benzene sulfonic acid, 1-naphthalene sulfonic acid, 1,5-naphthalenedisulfonic acid, sulphuric acid, triethylamine, N-methylmorfoline, potassium carbonate, diisopropylethylamine and pyridine. Preferable catalyst is PTSA or triethylamine.

This reaction is carried out in the presence of a catalyst, Suitable catalysts are PTSA, methane sulfonic acid, benzene sulfonic acid, sulphuric acid and triethylamine. Among them, PTSA or triethylamine are preferred. The amount of catalyst is not critical, and generally it can be from an equimolar amount of 1 to 1,5 with respect to the compound of formula (II).

The amount of acetate derivative is not critical, and generally it can be from an equimolar amount of 4,5 to 5,5 with respect to the compound of formula (II).

The acetylation reaction may take place at temperature from 15°C to 75°C. Preferably, from 20°C to 70°C.

Once the acetylation step is completed, the compound of formula (I) is isolated from the reaction medium by means of conventional methods.

And, optionally, preparing the pharmaceutically acceptable salts thereof by either:
d) converting Prasugrel base of formula (I) with maleic acid in the presence of a polar aprotic solvent such as acetone, methyl ethyl ketone or MIBK, preferably acetone, into Prasugrel maleate of formula (Ib) 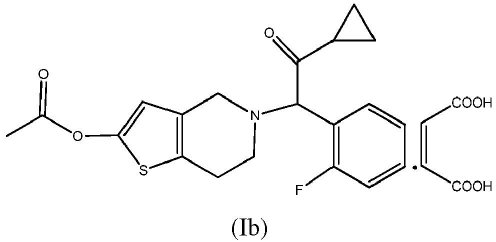 and, optionally, purifying prasugrel maleate, obtaining prasugrel base and, then converting prasugrel base into another pharmaceutically acceptable salt;

The reaction may be carried out mixing the compound of formula (I) with maleic acid at a temperature from 20 °C to 30 °C, preferably from 25 °C to 30 °C. The mixture is further cooled down to a temperature from -5 °C to 15 °C, preferably from 0 °C to 5 °C. The time required for the reaction may vary; however, the preferred period for conversion into maleate salt is from 2 to 4 hours. The yield obtained is around 90% and the purity is 99,2%.

And, alternatively, preparing the pharmaceutically acceptable salts Prasugrel by:
e) converting Prasugrel base of formula (I) into a pharmaceutically acceptable salts of prasugrel.

In an embodiment of the invention, Prasugrel maleate of formula (Ib) is purified by conventional methods that can be used at industrial scale such as solvent extraction, filtration, slurring, washing, phase separation, evaporation, centrifugation or crystallisation.

A suitable method for the purification of prasugrel maleate involves the preparation of prasugrel base by addition of water, an organic aprotic solvent and a base. The organic aprotic solvent is selected from ethyl acetate, methyl acetate, propyl acetate, isopropyl acetate, butyl acetate, isobutyl acetate, or toluene; the preferred solvents are ethyl acetate and toluene. Suitable bases are selected from carbonates, bicarbonates or ammonia derivates. The preferred bases are carbonates and bicarbonates, more preferably sodium carbonate. Afterwards, the generated prasugrel base is isolated by known industrial isolation methods such as extraction, decantation, crystallization or precipitation. When the isolation of prasugrel is carried out by extraction an aprotic solvent is used. Suitable aprotic solvents are ethyl acetate, methyl acetate, propyl acetate, isopropyl acetate, butyl acetate, isobutyl acetate, or toluene; the preferred solvents are ethyl acetate and toluene. Further, the prepared Prasugrel base (I) is purified. The purification may be carried out by washing with polar solvents selected from C1-C6 lineal or branched alcohols or nitriles. The preferred solvents are methanol, ethanol or acetonitrile.

Subsequently, purified prasugrel base is further converted into prasugrel maleate by addition of maleic acid in the presence of a polar aprotic solvent such as ketones, preferably acetone, methyl ethyl ketone or MIBK. Prasugrel maleate is obtained in high purity. Particularly, prasugrel maleate is obtained with a purity higher than 99.5%.

In another embodiment of the invention, the pharmaceutically acceptable salt of prasugruel is prepared by converting prasugrel base into the desired pharmaceutically acceptable salt, as depicted below: 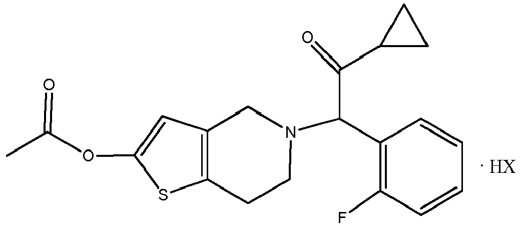 by adding a solution of the conjugated acid of the desired salt in a polar solvent.

Suitable conjugated acid is a conjugated acid of an inorganic or an organic acid, selected from maleic acid, oxalic acid, citric acid, and tartaric acid, sulfonic acid derivates and hydrohalic acid. Preferably, the conjugated acid is a hydrohalic acid.

Pharmaceutically acceptable salts of Prasugrel can be inorganic salts such as hydrochloride, hydrobromide, dihydroclhoride or hydrofluoride or organic salts such as oxalate, tartrate or citrate. Among them, hydrochloride salt of formula (Ia) is preferred.

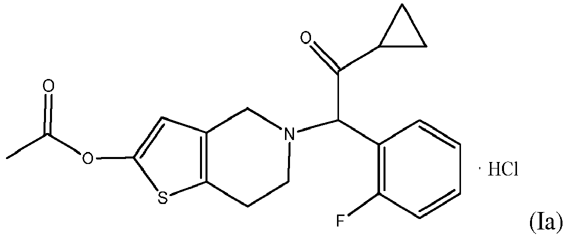

The preparation of pharmaceutically acceptable salts of prasugrel comprises the steps:
i) Preparation of prasugrel base by addition of a base and an aprotic solvent
ii) Isolation of prasugrel base by means of industrial isolation techniques such as extraction, decantation, crystallization or precipitation.
iii) Addition of the conjugated acid of the desired salt (HX) in a polar organic solvent to yield the desired salt of prasugrel.

Suitable bases used in step i) include metal hydroxides, such as sodium hydroxide and potassium hydroxide, metal carbonates, such as sodium carbonate and potassium bicarbonate, ammonia derivatives such as triethyl amine, dicyclohexylamine, N,N-diisopropylethylamine or methanolic ammonia. Preferably, the base is a carbonate, more preferably sodium carbonate.

Suitable aprotic solvents used in steps i) and ii) can be selected from: ethyl acetate, methyl acetate, propyl acetate, isopropyl acetate, butyl acetate, isobutyl acetate or toluene, preferably ethyl acetate or toluene.

One method for obtaining prasugrel hydrochloride involves the preparation of prasugrel base from prasugrel maleate, by adding a base and an aprotic solvent, followed by conversion of the prasugrel base into prasugrel hydrochloride by adding a solution HCl in a polar solvent or by adding to previous prepared prasugrel base a protic solvents or aprotic solvents in combination with at least one equivalent of a protic solvent compared to prasugrel and tumethylchlorosilane (TMS-Cl). Suitable protic solvents are: C1-C6 linear or branched alcohols, IPA. Preferable, the aprotic solvent is acetone.

Suitable bases used in the preparation of prasugrel base are selected from metal hydroxides, such as sodium hydroxide and potassium hydroxide, metal carbonates, such as sodium carbonate and potassium bicarbonate, ammonia derivatives such as triethyl amine, dicyclohexylamine, N,N-diisopropylethylamine or methanolic ammonia. Preferably, the base is a carbonate, more preferably sodium carbonate.

Suitable aprotic solvents used in the preparation of prasugrel base are selected from ethyl acetate, methyl acetate, propyl acetate, isopropyl acetate, butyl acetate, isobutyl acetate or toluene, preferably ethyl acetate or toluene.

The processes according to the present invention avoid the purification of the prepared products by means of chromatographic methods, which are not feasible at industrial scale.

The processes according to the present invention allow the preparation of pharmaceutical acceptable salts of prasugrel in high purity and yields.

The present invention also relates to a process for preparing a compound of formula (IV) 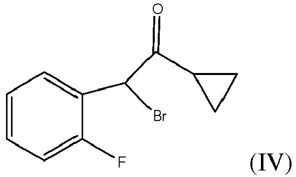 comprising a) bromination of the compound of formula (V) 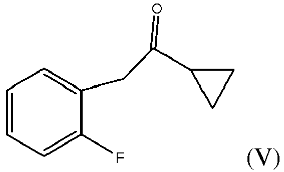 with liquid bromine in the presence of a polar protic solvent to obtain the compound of formula (IV).

This reaction is carried out at a reaction temperature ranging from 15 to 40°C, preferably from 20 to 32°C, most preferably from 23 to 30°C.

Suitable polar protic solvents are selected from alcohols, alkoxylated alcohols, aryloxylated alcohols and polyols. Preferable polar protic solvent is methanol.

The molar ratio of liquid bromine to compound of formula (V) is from 1 to 2, preferably, from 1.1 to 1.5.

The present invention also relates to the use of the compound of formula (IV) obtained according to first and second aspect of the present invention, for preparing prasugrel base and salts thereof.

The present invention is further illustrated by the following examples. They should in no case be interpreted as a limitation of the scope of the invention as defined in the claims.

### EXAMPLES

### Example 1. Preparation of 2-Fluoro-alpha-cyclopropylcarbonyl benzyl bromide

150 g of cyclopropyl-2-fluorobenzyl ketone and 1.8 l of methanol were mixed in a 10-liter 4-neck flask fitted with condenser. Subsequently, 147.9 g of liquid bromine were added dropwise to the mixture over a period of 3.5 hours, which was further stirred for 2.5 hours at 25-30 °C. Afterwards, the mixture was cooled down to 10 °C and 3.8 1 of pre chilled water added dropwise to the reactor. The reaction mass was extracted with 2.25 1 of ethyl acetate and the organic and the aqueous layer separated. The aqueous layer was extracted with 750 ml of ethyl acetate. Successively the layers were separated and the ethyl acetate layers were mixed and washed with 750 ml of a 10% w/v solution of sodium metabisulphite. The layers were separated and the ethyl acetate layer was washed with 750 ml of a 10% w/v solution of sodium bicarbonate.

Afterwards, the layers were separated and the ethyl acetate layer was washed with 750 ml of brine solution. Lastly, the ethyl acetate layer was separated and was dried over 100 g of sodium sulphate and concentrated under vacuum at 50-55 °C to yield 205 g of the title compound as a yellow coloured oil. Yield: 94.65%. Purity (HPLC): 95.76%

### Example 2. Preparation of 2-Fluoro-alpha-cyclopropylcarbonyl benzyl bromide

150 g of cyclopropyl-2-fluorobenzyl ketone and 1.8 l of methanol were mixed in a 10-liter 4-neck flask fitted with condenser. Next, 147.87 g of liquid bromine were added to the mixture dropwise over a period of 3.5 hours, which was further stirred for 2.5 hours at 25-30 °C. Afterwards, the mixture was cooled down to 10 °C and 3.8 1 of pre chilled water were added dropwise to the reactor. The reaction mass was extracted with 1.5 1 of toluene and the organic and the aqueous layer were separated. The aqueous layer was extracted with 750 ml of toluene. Successively, the layers were separated and toluene layers were mixed and washed with 750 ml of a 10% w/v aqueous solution of sodium metabisulphite. The layers were separated and the toluene layer washed with 750 ml of a 10% w/v solution of sodium bicarbonate. Next, the layers were separated and the toluene layer was washed with 750 ml of brine solution. Lastly, the layers were separated and the toluene layer was concentrated under vacuum at 50-55 °C to yield 203.5 g of the title compound, as a yellow coloured oil. Yield: 94.00%. Purity (HPLC): 94.06%

### Example 3. Preparation of 5-[2-Cyclopropyl-1-(2-fluoro-phenyl)-2-oxo-ethyl]-5,6,7,7a-tetrahydro-4H-thieno[3,2-c]pyridin-2-one

143.4 g of 5,6,7,7a-Tetrahydro-4H-thieno[3,2-c]pyridin-2-one hydrochloride and 1.434 1 of acetonitrile were mixed in a 5-liter 4-neck round bottom flask. The mixture was stirred for 10 minutes and subsequently cooled down to -5-0 °C. Afterwards, 175 g of 2-Fluoro-alpha-cyclopropylcarbonyl benzyl bromide were added to the reactor and further stirred for 10 minutes. Next, 145 ml of a methanolic ammonia solution (16% w/v) were added to the mixture dropwise over a period of 2 hours and further stirred for 2 hours. After, an additional amount of methanolic ammonia solution, 42 ml, was added dropwise over a period of 1 hour. Afterwards, the mixture was quenched in 2.65 1 of water and 35 ml of concentrated HCl. Successively, the pH of the mixture was adjusted to 7.0 with 175 ml of a 10% (w/v) solution of sodium bicarbonate. The reaction mass was extracted with 1.75 1 of ethyl acetate. The layers were separated and the aqueous layer was extracted with 875 ml of ethyl acetate. Both ethyl acetate layers were mixed, washed with 585 ml of brine solution and dried with 100 g of sodium sulphate. Lastly, the ethyl acetate layer was distilled out under vacuum at 50-55 °C to give 214 g of the title compound, as a brown coloured semi solid. Yield: 95.00%. Purity (HPLC): 83.04%

### Example 4. Preparation of 5-[2-Cyclopropyl-1-(2-fluoro-phenyl)-2-oxo-ethyl]-5,6,7,7a-tetrahydro-4H-thieno[3,2-c]pyridin-2-one

84.01 g of 5,6,7,7a-Tetrahydro-4H-thieno[3,2-c]pyridin-2-one hydrochloride and 0.84 1 of acetonitrile were mixed in a 2 liter 4 neck round bottom flask. The mixture was cooled down to -5-0 °C. In addition, 102.5 g of 2-Fluoro-alpha-cyclopropylcarbonyl benzyl bromide were added to the reactor and it was further stirred for 10 minutes at - 5-0 °C. Next, 84.8 ml of a methanolic ammonia solution (16% w/v) were added dropwise to the mixture over a period of 2 hours and it was further stirred for 1 hour. Subsequently, 24.4 ml more of methanolic ammonia solution were added dropwise over a period of 1 hour. Afterwards, the mixture was quenched in 1.556 1 of water and 20.55 ml of concentrated HCl. Next, the pH of the mixture was adjusted to 7.0 with 102.7ml of a 10% (w/v) solution of sodium bicarbonate. The reaction mass was extracted with 1.027 1 of ethyl acetate. The aqueous layer was extracted with 513 ml of ethyl acetate. The layers were separated. Both ethyl acetate layers were mixed, washed with 345ml of brine solution and dried with 75 g of sodium sulphate. Lastly, the ethyl acetate layer was distilled out under vacuum at 50-55 °C to give 125g of the title compound as a brown coloured semi solid. Yield: 95.00%. Purity (HPLC): 88.6%

### Example 5. Preparation of 5-[2-Cyclopropyl-1-(2-fluoro-phenyl)-2-oxo-ethyl]-5,6,7,7a-tetrahydro-4H-thieno[3,2-c]pyridin-2-one

143.4 g of 5,6,7,7a-Tetrahydro-4H-thieno[3,2-c]pyridin-2-one hydrochloride were mixed in a 5 liter 4 neck round bottom flask with 1434 ml of acetonitrile. The mixture was stirred for 10 minutes and subsequently cooled down to -5-0 °C. In addition, 175 g of 2-Fluoro-alpha-cyclopropylcarbonyl benzyl bromide were added to the reactor and further stirred for 10 minutes. Next, 145ml of methanolic ammonia solution (16% w/v) were added dropwise to the mixture over a period of 2 hours and further stirred for 2 hours. An additional amount of 42 ml of the methanolic ammonia solution were added dropwise over a period of 1 hour. Afterwards, the mixture was quenched in 2.65 1 of water and 35 ml of concentrated HCl. After that, the pH of the mixture was adjusted to 7.0 with 175 ml of a 10% (w/v) solution of sodium bicarbonate. The reaction mass was extracted with 1.75 1 of toluene. The layers were separated and the aqueous layer was extracted with 875 ml of toluene. Both toluene layers were mixed and washed with 585 ml of brine solution. Lastly, the solvent was distilled out under vacuum at 50-55 °C to give 211 g of the title compound, as a brown coloured semi solid. Yield: 93.60%. Purity (HPLC): 82.70%

### Example 6. Preparation of 5-[2-Cyclopropyl-1-(2-fluoro-phenyl)-2-oxo-ethyl]-5,6,7,7a-tetrahydro-4H-thieno[3,2-c]pyridin-2-one

20.4 g of 5,6,7,7a-Tetrahydro-4H-thieno[3,2-c]pyridin-2-one hydrochloride 205 ml of acetonitrile were mixed in a 1 liter 4 neck round bottom flask with. The mixture was stirred for 10 minutes and subsequently cooled down to -5-0 °C. In addition, 25 g of 2-Fluoro-alpha-cyclopropylcarbonyl benzyl bromide were added to the reactor and further stirred for 10 minutes. Next, 25.4 ml of triethylamine were added dropwise to the mixture and further stirred for 1 hour. Afterwards, the mixture was quenched in 378 ml of water and 5 ml of concentrated HCl. After that, the pH of the mixture was adjusted to 7.0 with 21 ml of a 10% (w/v) solution of sodium bicarbonate. The reaction mass was extracted with 250 ml of ethyl acetate, the layers were separated and the aqueous layer was extracted with 125 ml more of ethyl acetate. Both ethyl acetate layers were mixed, washed with 85 ml of brine solution and dried with 15 g of sodium sulphate. Lastly, the solvent was distilled out under vacuum at 50-55 °C to give 30.5 g of the title compound as a brown colour semi solid. Yield: 94.70%. Purity (HPLC): 54.40%

### Example 7. Preparation of 5-(2-cyclopropyl-1-(2-fluorophenyl)-2-oxoethyl)-5,6,7,7a-tetrahydro-4H-thieno[3,2-c]pyridin-2-one

12 g of sodium carbonate (113 mmol) and 9.7 g of 5,6,7,7a-tetrahydro-4H-thieno[3,2-c]pyridin-2-one hydrochloride (50.6 mmol) were added to a solution of 13 g of 2-fluoro-alpha-cyclopropylcarbonyl benzyl bromide (50.6 mmol) in 40 mL of methyl ethyl ketone at 25 °C. The resulting reaction mixture was stirred for 16 hours at 25°C. The solid by-product was filtered off, and the mother liquor containing the compound 5-(2-cyclopropyl-1-(2-fluorophenyl)-2-oxoethyl)-5,6,7,7a-tetrahydro-4H-thieno[3,2-c]pyridin-2-one was separated and further treated. Compound 5-(2-cyclopropyl-1-(2-fluorophenyl)-2-oxoethyl)-5,6,7,7a-tetrahydro-4H-thieno[3,2-c]pyridin-2-one was isolated by removing the solvent at reduced pressure to yield 13.4 g of the title compound, as brown oil. Yield 80 %. Purity (HPLC) 84 %.

### Example 8. Preparation of 5-(2-cyclopropyl-1-(2-fluorophenyl)-2-oxoethyl)-4,5,6,7-tetrahydro thieno[3,2-c]pyridin-2-yl acetate (prasugel base).

14.8 mL of triethylamine (100 mmol) and 10 mL of acetic anhydride (100 mmol) were added to a solution of 10 g of 5-(2-cyclopropyl-1-(2-fluorophenyl)-2-oxoethyl)-5,6,7,7a-tetrahydro-4H-thieno[3,2-c]pyridin-2-one (30 mmol) in 50 mL of methylene chloride at 25°C. The resulting reaction mixture was stirred for 1 hour at 25°C. Afterwards, 100 mL of a 5 % (w/v) aqueous solution of sodium bicarbonate was charged to the reaction mixture and stirred for 15 minutes. The organic layer was washed with 100 mL of a 5 % (w/v) aqueous solution of sodium bicarbonate and the organic solvent removed under reduced pressure to obtain 10 g of the title compound, as a colourless oil. Finally, 30 mL of methanol were added to the colourless oily and stirred for 1 hour at 25 °C. The resulting suspension was cooled down to 0 °C and stirred for 1 hour. The solid was filtered and washed with 10 mL of cool methanol and dried at 40°C for 16 hours yielding 5.9 g of the title compound. Yield 54 %. Purity (HPLC) 99 %.

### Example 9. Preparation of 5-(2-cyclopropyl-1-(2-fluorophenyl)-2-oxoethyl)-4,5,6,7-tetrahydro thieno[3,2-c]pyridin-2-yl acetate (prasugel base).

14.8 mL of triethylamine (100 mmol) and 10 mL of acetic anhydride (100 mmol) were added to a solution of 10 g of 5-(2-cyclopropyl-1-(2-fluorophenyl)-2-oxoethyl)-5,6,7,7a-tetrahydro-4H-thieno[3,2-c]pyridin-2-one (30 mmol) in 50 mL of toluene at 25°C. The resulting reaction mixture was stirred for 1 hour at 25 °C and 100 mL of a 5 % (w/v) aqueous solution of sodium bicarbonate were further added to the mixture and stirred for 15 minutes. The organic layer was washed with 100 mL of a solution of 5 % (w/v) aqueous solution sodium bicarbonate and the organic solvent was removed under reduced pressure to obtain 10 g of the title compound, as colourless oil. Finally, 30 mL of methanol were added to the oily residue and stirred for 1 hour at 25 °C. The resulting suspension was cooled down to 0 °C and stirred for 1 hour. The solid was filtered and washed with 10 mL of cool methanol and dried at 40°C for 16 hours to yield 5.8 g of the title compound. Yield 53 %. Purity (HPLC) 99 %.

### Example 10. Preparation of 2-acetoxy-5-(alpha-cyclopropyl carbonyl-2-fluorobenzyl)-4,5,6,7 tetrahydrothieno [3,2-c] pyridine (Prasugrel base)

349 ml of isopropenyl acetate, 119,4 g of PTSA and 208 g of 5-[2-Cyclopropyl-1-(2-fluoro-phenyl)-2-oxo-ethyl]-5,6,7,7a-tetrahydro-4H-thieno[3,2-c]pyridin-2-one were mixed in a 2 liter round bottom flask fitted with a condenser, The mixture was heated up to 65-70 °C and stirred at this temperature for 3 and a half hours. Afterwards, the reaction mass was cooled down to 25-30 °C and quenched with 2.1 l of chilled water. In addition, 160 g of sodium carbonate dissolved in 500 ml of water were added to adjust the pH of the reaction mass to 6.5 - 7. The mixture was extracted with 2 1 of ethyl acetate and the layers were separated. The aqueous layer was further extracted with 1 l of ethyl acetate and the layers were separated. Subsequently, both organic layers obtained from the extractions were mixed and washed with 1 l of brine solution. The layers were separated and the organic layer was subsequently dried with 100 g of sodium sulphate. The ethyl acetate was distilled out under vacuum at 45-50 °C and 416ml of methanol were added to the mixture followed by stirring for 30 minutes. The obtained solid was filtered and dried at 60-65 °C for 30 minutes to yield 140 g of the title compound, as a cream coloured solid. Yield: 59.72%. Purity (HPLC): 98.73%

### Example 11. Preparation of 2-acetoxy-5-(alpha-cyclopropyl carbonyl-2-fluorobenzyl)-4,5,6,7 tetrahydrothieno [3,2-c] pyridine (Prasugrel base)

349 ml of isopropenyl acetate, 119,4g of PTSA and 208g of 5-[2-Cyclopropyl-1-(2-fluoro-phenyl)-2-oxo-ethyl]-5,6,7,7a-tetrahydro-4H-thieno[3,2-c]pyridin-2-one were mixed in a 2 liter round bottom flask fitted with a condenser. The mixture was heated up to 65-70 °C and stirred at this temperature for 3.5 hours. Afterwards, the reaction was cooled down to 25-30 °C and quenched with 2.1 l of chilled water. Next, 160 g of sodium carbonate dissolved in 500 ml of water were added to adjust the pH of the reaction mass to 6.5 - 7. 2 1 of toluene were added to the mixture and the layers were separated. The aqueous layer was extracted again with 1 l of toluene. Both organic layers obtained from the previous extractions were mixed and washed with 1 l of brine solution. Afterwards, the layers were separated and the toluene layer was concentrated under vacuum at 50-55 °C and 416 ml of methanol were added to the mixture and further stirred for 30minutes. Lastly, the solid was separated out, filtered and washed with 200 ml of methanol to yield 119.5 g of the title compound, as a cream coloured solid. Yield: 51.00%. Purity (HPLC): 98.73%

### Example 12. Preparation of prasugrel maleate salt

9.8 g of maleic acid and 120 ml of acetone were mixed in a reactor. The mixture was stirred for 15 minutes. Next, 30 g of Prasugrel base were added to the reactor and the solution was further stirred for 3h at 25-30 °C. A precipitate was formed after the stirring. Afterwards, the mixture was cooled down to 0-5 °C and further stirred for 1h. The mixture was filtered and the obtained solid was dried at 60-65 °C to yield 35 g of prasugrel maleate salt. Yield: 89%. Purity(HPLC): 99.16%.

### Example 13. Preparation of prasugrel maleate salt

50.3g of maleic acid and 620 ml of acetone were mixed in a 1 liter round bottom flask. The mixture was stirred for 5 minutes. Next, 155 g of 2-acetoxy-5-(alpha-cyclopropyl carbonyl-2-fluorobenzyl)-4,5,6,7 tetrahydrothieno [3,2-c] pyridine were added to the reactor and the solution was further stirred for 3h at 25-30 °C. A precipitate was formed after the stirring. Afterwards, the mixture was cooled down to 0-5 °C and further stirred for 1h. The mixture was filtered, washed with 310ml of acetone and the obtained solid dried at 60-65 °C to yield 182.56 g of prasugrel maleate salt. Yield: 89.84%. Purity(HPLC): 99.20%.

### Example 14. Preparation of Prasugrel base pure

180 g of prasugrel maleate, 1.8 1 of water and 1.8 1 of ethyl acetate were charged in a 10 litre round bottom flask. The pH of the mixture was adjusted to 6.8 by adding a solution of 38.92 g of sodium carbonate dissolved in 389 ml of water. The mixture was further stirred during 15 minutes. The two different layers formed were separated. Afterwards, the aqueous layer was extracted with 360 ml of ethyl acetate and the layers were separated. Subsequently, both ethyl acetate layers were combined and washed with 370 ml of a 20% w/v aqueous solution of sodium chloride. The ethyl acetate layer was next dried over 50 g of sodium sulphate and the solvent was distilled out under vacuum at 50-55 °C. After the distillation, 540 ml of methyl cyclohexane were added to the residue and it was further stirred for 30 minutes. The obtained solid was filtered and mixed with 1.54 1 of methanol heated up to 60-65 °C and stirred for 15 minutes. Afterwards, the reaction mass was cooled down to 25-30 °C and was maintained at the same temperature for 1 hour. The solid was filtered and washed with 255 ml of methanol. Lastly, the mixture was dried at 60-65 °C for 5-6 hours under vacuum to yield 94.0 g of prasugrel base pure. Yield: 68.5%. Purity (HPLC): 99.6%.

### Example 15. Preparation of Prasugrel base pure

180 g of prasugrel maleate, 1.8 1 of water and 1.8 1 of toluene were charged in a 10 litre round bottom flask. The pH of the mixture was adjusted to 6.8 by adding a solution of 38.92 g of sodium carbonate dissolved in 389 ml of water. The mixture was further stirred during 15 minutes. The two different layers formed were separated. Afterwards, the aqueous layer was extracted with 360 ml of toluene. Subsequently, both toluene layers were combined and washed with 370 ml of a 20% w/v aqueous solution of sodium chloride. Next, toluene was distilled out under vacuum at 50-55 °C. After the distillation, 540 ml of methyl cyclohexane were added to the residue and it was further stirred for 30 minutes. The obtained solid was filtered. Afterwards, the solid was mixed with 1.54 l of methanol and was heated up to 60-65 °C and stirred for 15 minutes. Next, the reaction mass was cooled down to 25-30 °C and was maintained at the same temperature for 1 hour. The solid was filtered and washed with 255 ml of methanol. Lastly, the mixture was dried at 60-65 °C for 5-6 hours under vacuum to yield 93.0g of prasugrel base pure. Yield: 67.7%. Purity (HPLC): 99.6%.

### Example 16. Preparation of Prasugrel base pure

12 g of Prasugrel base were mixed with 120ml of methanol. The reaction was heated up to 65 °C and stirred for 30 minutes. Afterwards, the mixture was cooled down to 25-30 °C and further stirred for 1hour. The reaction mass was filtered and dried at 60-65 °C to yield 7.8 g of prasugrel base pure. Yield: 65%. Purity (HPLC): 99.65%.

### Example 17. Preparation of Prasugrel maleate pure

14.6 g of maleic acid were mixed with 180ml of acetone in a 1 lit round bottom flask and the mixture was stirred for 5 minutes. 45 g of prasugrel base pure were added to the reactor and the mixture was further stirred for 3 hours at room temperature. Afterwards, the mixture was cooled down to 0 - 5 °C. Next, the reaction mass was filtered, washed with 90 ml of acetone. The solid obtained was dried under vacuum at 50-55 °C for 6-8 hours to yield 52.9 g of prasugrel maleate pure. Yield: 89.84%. Purity (HPLC): 99.75%.

### Example 18. Preparation of Prasugrel hydrochloride

30 g of prasugrel base pure were mixed with 300ml of acetone and 10.66 ml of TMS-Cl. The mixture was heated up to 45 °C and 0.16 ml of water were added. Afterwards, the mixture was cooled down to 0-5 °C and stirred for 1h. The mixture was filtered. The solid obtained was dried at 60-65 °C for 4 hours to yield 30.2 g of prasugrel hydrochloride. Yield: 91.72%. Purity (HPLC): 99.80%.

## Claims

1. Process for preparing Prasugrel and, optionally, pharmaceutically acceptable salts thereof, comprising the following steps:
a) bromination of the compound of formula (V) 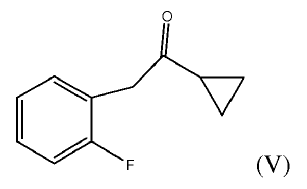 with liquid bromine in the presence of a polar protic solvent to obtain the compound of formula (IV), wherein the reaction temperature range is from 15 to 40 °C 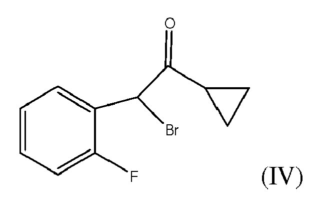
b) condensing the compound of formula (IV) with the compound of formula (III) 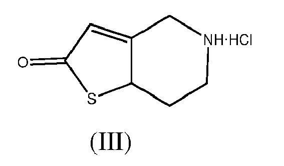 in the presence of a base in a polar aprotic solvent to obtain the compound of formula (II) 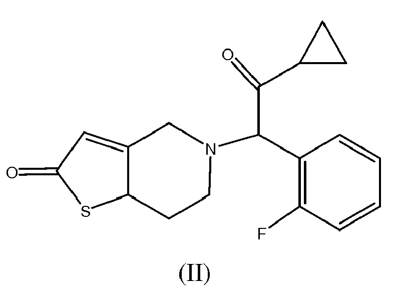
c) acetylating the compound of formula (II) in the presence of an aprotic solvent, an acetylating agent and a catalyst to obtain Prasugrel base of formula (I), 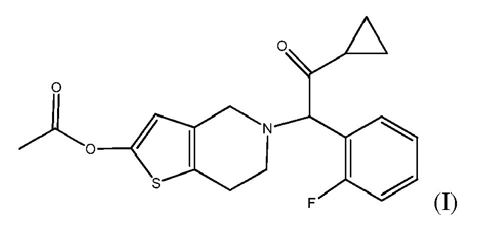
and, optionally, preparing pharmaceutically acceptable salts thereof by either:
d) converting Prasugrel base of formula (I) with maleic acid in the presence of a polar aprotic solvent into Prasugrel maleate of formula (Ib) 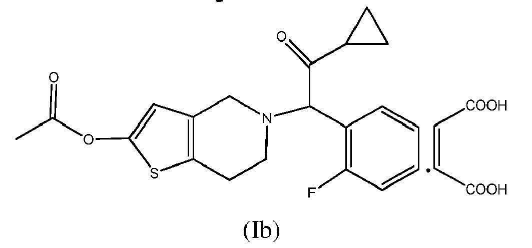 and, optionally, purifying prasugrel maleate, obtaining prasugrel base and, then converting prasugrel base into another pharmaceutically acceptable salt;
or either
e) converting Prasugrel base of formula (I) into a pharmaceutically acceptable salt of prasugrel.

2. The process according to claim 1, wherein in the step a) the reaction temperature range is from 20 to 32°C, most preferably from 23 to 30 °C.

3. The process according to claim 1 to 2, wherein in the step a) the polar protic solvent is selected from alcohols, alkoxylated alcohols, aryloxylated alcohols and polyols.

4. The process according to claim 3, wherein in the step a) the alcohol is methanol.

5. The process according to any preceding claims, wherein in the step a) the molar ratio of liquid bromine to compound of formula (V) is from 1 to 2, preferably, from 1.1 to 1.5.

6. Process according to any preceding claims, wherein in the step b) the base is selected from metal hydroxides, metal carbonates and ammonia derivatives, preferably sodium carbonate or methanolic ammonia.

7. Process according to any preceding claims, wherein in the step b) the reaction is carried out at a temperature range from -10 to 75°C. When the base is an ammonia derivate the reaction is carried out at a temperature range from -5 to 0°C and when the base is metal carbonate the reaction is carried out at a temperature range from 15 to 30°C.

8. Process according to claim 6, wherein in the step b) the reaction is carried out at 6≤pH≤8, preferably, at pH 7.

9. Process according to any preceding claims, wherein in the step c) the acetylating agent is selected from acetic anhydride, acetyl halides or acetates, preferably the acetylating agent is isopropenyl acetate, acetyl chloride, acetic anhydride or methyl acetate.

10. Process according to any preceding claims, wherein in the step c) the catalyst is selected from PTSA, methane sulfonic acid, triethylamine, benzene sulfonic acid and sulfuric acid, preferably PTSA or triethylamine.

11. Process according to any preceding claims, wherein the pharmaceutically acceptable salt of Prasugrel is prepared by converting Prasugrel base into the desired pharmaceutically acceptable salt of prasugrel HX 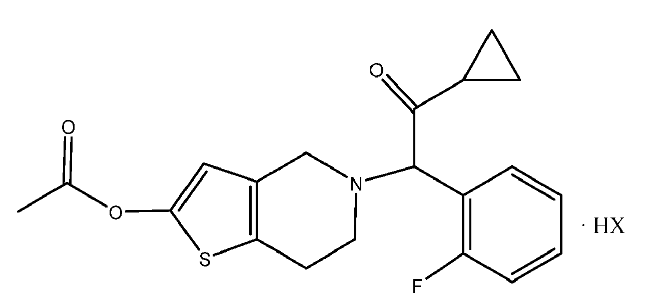 by adding a solution of the conjugated acid of the desired salt in a polar solvent.

12. Process according to claim 9, wherein said conjugated acid is selected from the conjugated acid of an organic acid selected from maleic acid, oxalic acid, citric acid, and tartaric acid and sulfonic acid derivates, preferably a hydrohalic acid.

13. Process according to claim 11, wherein being HCl the conjugated acid, the Prasugrel hydrochloride (Ia) 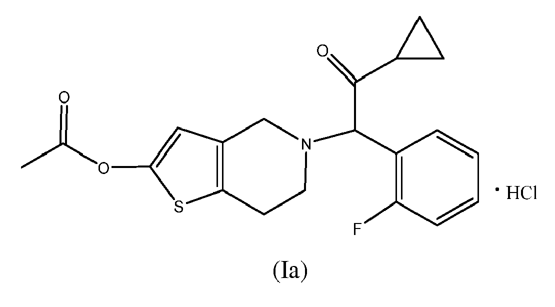 is obtained by either adding a solution of HCl in a polar solvent or by adding to previous prepared prasugrel base a protic solvent or an aprotic solvent in combination with at least one equivalent of a protic solvent compared to prasugrel and tumethylchlorosilane (TMS-Cl).

14. Process according to claim 13, wherein the aprotic solvent is acetone.

15. Process according to any preceding claims, wherein prasugrel maleate (Ib) is converted into prasugrel base by adding a base and an aprotic polar solvent.

16. Process for preparing a compound of formula (IV) 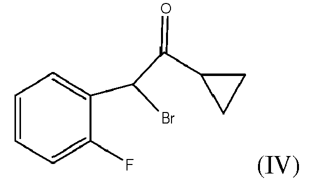 comprising a) bromination of the compound of formula (V) 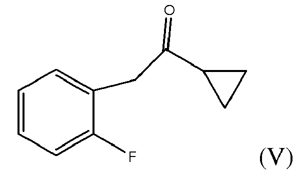 with liquid bromine in the presence of a polar protic solvent to obtain the compound of formula (IV), wherein the reaction temperature range is from 15 to 40 °C.

17. The process according to claim 16, wherein the reaction temperature range is from 20 to 32°C, most preferably from 23 to 30 °C.

18. The process according to claims 16 or 17, wherein the molar ratio of liquid bromine to compound of formula (V) is from 1 to 2, preferably, from 1.1 to 1.5.

19. Use of the compound of formula (IV) obtained according to any claims 16 to 18, for preparing prasugrel base ant salts thereof.
